**Europäisches Patentamt**

**European Patent Office**

**Office européen des brevets**

(11) Veröffentlichungsnummer: **0 183 253 B1**

# (12) EUROPÄISCHE PATENTSCHRIFT

(45) Veröffentlichungstag der Patentschrift: **04.09.91**

(51) Int. Cl.⁵: **A61K 35/38**, A61K 35/26

(21) Anmeldenummer: **85115084.7**

(22) Anmeldetag: **28.11.85**

Die Akte enthält technische Angaben, die nach
dem Eingang der Anmeldung eingereicht wurden
und die nicht in dieser Patentschrift enthalten sind.

(54) **Arzneimittel enthaltend einen aus den Peyer'schen Plaques hergestellten Organextrakt zur Behandlung bei Diabetes Mellitus und Pankreatitis.**

(30) Priorität: **28.11.84 DE 3443411**

(43) Veröffentlichungstag der Anmeldung:
**04.06.86 Patentblatt 86/23**

(45) Bekanntmachung des Hinweises auf die
Patenterteilung:
**04.09.91 Patentblatt 91/36**

(84) Benannte Vertragsstaaten:
**AT CH DE FR LI**

(56) Entgegenhaltungen:
**EP-A- 0 049 379**

(73) Patentinhaber: **Zoubek, Eugen, Dr.**
**Allescherstrasse 2a**
**W-8000 München 71(DE)**

(72) Erfinder: **Zoubek, Eugen, Dr.**
**Allescherstrasse 2a**
**W-8000 München 71(DE)**

(74) Vertreter: **Tetzner, Volkmar, Dr.-Ing. Dr. jur.**
**Van-Gogh-Strasse 3**
**W-8000 München 71(DE)**

**Beschreibung**

Die Erfindung betrifft die Verwendung eines aus den Peyer'schen Plaques hergestellten Organextraktes zur Herstellung eines Arzneimittels.

Durch die EP-A-0 049 379 ist bereits ein Verfahren zur Herstellung eines die Stimulation der Proliferationsrate von Leberzellen bewirkenden Faktors bekannt, bei dem die Peyer'schen Drüsen von Versuchstieren homogenisiert, auf einen pH-Wert von 5,5 gebracht, bei 95°C Hitze denaturiert und zentrifugiert werden, wobei der Überstand den Faktor enthält.

Der Erfindung liegt die Aufgabe zugrunde, eine weitere therapeutische Anwendung eines aus den Peyer'schen Plaques hergestellten Organextraktes anzugeben.

Diese Aufgabe wird erfindungsgemäß durch die Verwendung eines aus den Peyer'schen Plaques hergestellten Organextraktes zur Herstellung eines Arzneimittels zur unterstützenden Behandlung bei Diabetes mellitus sowie zur Behandlung von Pankreatitis gelöst.

Eine vorteilhafte weitere Ausgestaltung der Erfindung sieht die Verwendung eines solchen aus den Peyer'schen Plaques hergestellten Organextraktes in Verbindung mit Thymus-Organextrakt vor.

Wie sich bei den der Erfindung zugrundeliegenden Versuchen zeigte, stehen nicht nur die Thymusdrüse, sondern auch die Peyer'schen Plaques in engem Zusammenhang mit dem Immunsystem. Dabei sind die Peyer'schen Plaques am Aufbau des humoralen Immunsystems maßgeblich beteiligt.

Die spezifischen Phänomene der Immunität werden durch Immunozyten vermittelt. Es sind dies Zellen, die spezifisch mit Antigen reagieren. Zum Zellsystem der Immunozyten gehören T- und B-Lymphozyten, die beide aus den Knochenmarks-Stammzellen hervorgehen. Die T-Lymphozyten reifen dabei durch den Einfluß der Thymusfaktoren heran und werden zu Untergruppen mit unterschiedlichen funktionellen Steuerungsaufgaben im Immungeschehen differenziert.

So wie die T-Lymphozyten durch den hormonellen Einfluß der Thymusdrüse ihre Immunkompetenz für die zelluläre Abwehr erhalten, empfangen die B-Lymphozyten unter dem Einfluß hormoneller Faktoren der Peyer'schen Plaques ihre Immunkompetenz für die humorale Abwehr.

Im gesamten Magen-Darm-Trakt (von der Mundhöhle bis zum Enddarm) finden sich bei Mensch und Säugetieren gruppierte Anhäufungen von Lymphknoten oder einzelne Lymphknötchen. Eine besondere Anhäufung findet man in der Mundhöhle im Bereich des WALDEYER'schen Lymphatischen Rachenringes, im Bereich des Dünndarms in den Peyer'schen Plaques und in der Appendix.

Die Peyer'schen Plaques werden heute auch als "darmassoziiertes Lymphgewebe" bezeichnet. Es werden in diesen histologischen Bereichen alle Formen weißer Blutkörperchen, besonders auch T- und B-Lymphozyten gefunden. Der relative Anteil von T- und B-Lymphozyten hängt von der jeweiligen antigenen Stimulation ab.

Rezirkulierende T- und B-Lymphozyten können über postkapilläre Venolen aus der Blutbahn in die Peyer'schen Plaques einwandern. Dabei enthalten die Peyer'schen Plaques antigensensitive Zellen, aus denen vornehmlich Ig A- aber auch Ig M- und Ig G- produzierende Plasmazellen entstehen können. In dem darmassoziierten Lymphgewebe finden sich neben Antikörpern der Klassen IgM, IgG, IgA und IgD vor allem sekretorische IgA (sIgA)-und auch sekretorische IgM (sIgM)-Antikörper.

Die immunologische Schleimhautbarriere wird durch humorale und zelluläre Immunreaktionen aufrechterhalten. Die Peyer'schen Plaques besiedeln nicht nur die intestinale Lamina propria mit IgA-Immunozyten, sondern es wandern auch IgA-enthaltende Zellen aus dem darmassoziierten Lymphgewebe in die Bronchialschleimhaut ein und umgekehrt. Ebenso findet eine Wanderung von Zellen aus dem Bronchialsystem in die Lamina propria des Darmes statt.

Diese und andere Befunde unterstreichen die nicht nur periphere Bedeutung der Peyer'schen Plaques, sondern lassen dieser relativ großen Lymphknotenansammlung auch zentrale und immunregulatorische Aufgaben für den gesamten Organismus zukommen. Man kann aus diesen Untersuchungen schließen, daß eine bemerkenswerte Wanderung von Immunoblasten zwischen den verschiedenen Schleimhäuten und Drüsen stattfindet, was das Konzept eines allgemeinen schleimhaut-assoziierten Immunsystems weiter stützt.

Das humorale Immunsystem hat dabei seinen Sitz wohl in den Peyer'schen Plaques, der Milz und in den Lymphknoten (diese Organe sind wohl als Äquivalent der bei den Vögeln vorhandenen bursa fabricii anzusehen). Auffallend ist eine morphologische Ähnlichkeit der Peyer'schen Plaques mit der bursa fabricii der Vögel. Es ist dies der Ort, wo die Prae-Lymphozyten aus dem Knochenmark zu den B-Effektor-Lymphozyten heranreifen. Diese bilden auf Antigenreize Antikörper in Form von Immunglobulinen. Dabei wird das Antigen in der Regel durch Makrophagen repräsentiert, wobei Thymus-Helferzellen unterstützend in den Prozeß der humoralen Abwehr eingreifen.

Wie die der vorliegenden Erfindung zugrundeliegenden Versuche zeigten, werden in den Peyer'schen Plaques noch andere Faktoren von hormonartiger Struktur produziert, die nicht nur auf die humorale Abwehr, sondern auf den gesamten Stoffwechsel einen bedeutenden Einfluß ausüben. So hat sich insbesondere gezeigt, daß Wirkstoffe aus den Peyer'schen Drüsen einen erheblichen Einfluß auf die resorptive Tätigkeit des Dünndarmes haben.

Ein Arzneimittel in Form eines aus den Peyer'schen Plaques hergestellten Organextraktes läßt sich nach dem durch die Erfindung bisher erreichten Kenntnisstand vorteilhaft für die Behandlung von Pankreatitis und zur unterstützenden Behandlung bei Diabetes mellitus verwenden.

Als besonders vorteilhaft hat sich auch die Verwendung eines aus den Peyer'schen Plaques hergestellten Organextraktes in Verbindung mit Thymus-Organextrakt erwiesen. Diese vorteilhafte Wirkung dürfte sich wie folgt erklären lassen:

Nach Brunner gibt es drei Mechanismen, die den immunologischen Regulationsmechanismus bewirken:
- die Immunreaktion der T-Lymphozyten, die durch ihre zytotoxische Eigenschaft gegeben ist,
- die Immunreaktion der IgG-Antikörper auf die Tumorzellen, welche dann durch die B-Lymphozyten zerstört werden,
- die Immunreaktion durch die Makrophagen, welche eine spezifische zytotoxische Wirkung auf Tumorzellen haben und von chemischen Botenstoffen, den Lymphokinen, freigesetzt werden, wobei die Makrophagen sowohl mit den T- als auch mit den B-Lymphozyten in Verbindung stehen.

Anzustreben ist nun therapeutisch, daß das humorale und das zelluläre Abwehrsystem gleichzeitig voll wirksam sind. Zu diesem Zweck müssen alle Immunfaktoren, d.h. die T- und die B-Lymphozyten, aktiviert werden. Gleichzeitig sollten natürlich alle immunsuppressiven Maßnahmen ausgeschaltet werden.

Bei intakter humoraler Abwehr gelingt es dem Organismus, mit den Homotoxinen fertig zu werden und durch Ausheilung einer Entzündung eine Entgiftung des Organismus durchzuführen, ohne daß Fermentschäden oder sonstige Laesionen an der Zelle entstehen.

Ein nach dem Sandberg'schen Verfahren aus den Peyer'schen Plaques hergestellter Organextrakt (im folgenden als PPX-Z Extrakt bezeichnet) wurde analytisch und präparativ in den Laboratorien der Universitätsklinik Frankfurt/Main untersucht. Der verwendete PPX-Z Extrakt wurde als Frischdrüsengesamtextrakt aus dem Dünndarm von ausgewählten Kälbern biologischer Aufzucht hergestellt.

Durchgeführt wurde u.a. eine SDS-Gradientengel-Polyacrylamid-Elektrophorese. In einem 10%-igen Polyacrylamid-Gel wurden bei einer Auftragsmenge von je 40 µl Probenvolumen mit einem diskontinuierlichen Tris-Glycin-puffer bei einer Trennzeit von 4 Stunden in einer Vertikal-Elektrophoresekammer der Firma Biorad neben menschlichen Serumproben gesunder Probanden und Dünndarmgewebe-Proben eines männlichen Schafsfoeten zum Vergleich der PPX-Z Extrakt in mehreren Untersuchungsreihen analytisch und teilweise auch präparativ untersucht.

Als Molekulargewichtsmarker wurden in den analytischen Trennungen folgende Reinsubstanzen verwendet:

| Ovotransferrin | MG | 76 000 – 78 000 Dalton |
|---|---|---|
| Albumin | " | 66 250 " |
| Ovalbumin | " | 45 000 " |
| Carboanhydrase | " | 30 000 " |
| Myoglobin | " | 17 200 " |

Es wurden in den angewandten Methoden der diskontinuierlichen SDS-Gradientengel-Polyacrylamid-Elektrophorese sowohl im PPX-Z Extrakt als auch in den mit untersuchten Vergleichsproben Proteinfraktionen im Bereich molarer Massen zwischen 76 000 und 2 500 Dalton gefunden, im einzelnen 18 Fraktionen zwischen 76 000 und 8 000 Dalton, reine Proteine und Glykoproteine, darüber hinaus aber noch mindestens weitere sechs Proteinfraktionen im Bereich unter 10 000 Dalton.

Durchgeführt wurden ferner klinisch-chemische, klinisch-biochemische und immunologische Untersuchungen zur Wirksamkeit des PPX-Z Extraktes.

Anerkannte Laborverfahren zur immunmodulatorischen Wirkung des PPX-Z Extraktes ergaben eine gesicherte Wirksamkeit auf Monozyten- bzw. Makrophagenfunktion, auf die T- und B-Zellen-vermittelte Immunantwort und Hinweise auf eine zusätzlich stimulierende Wirkung auf Anzahl und Funktion der Natural-Killer-Zellen (NK).

Folgende Nachweisuntersuchungen kamen zur Anwendung und ergaben alle in der kritischen Wertung eine immunmodulatorische Wirkung des PPX-Z Extraktes:
- In vivo Teste:
  Recall-Antigen-Test Byk-Merieux,
  Carbon-Clearance-Test an der Maus nach BIOZZI modif.
- In vitro-Teste (betreffend zelluläre Immunantwort):
  Spontan-Rosettentest mit Schafserythrozyten,
  Granulozytentest in der Modifikation nach BRAND,
  T-Zellen-Transformationstest nach WEIR,
  Makrophagen-Migrations-Inhibitionstest,
  Lymphozyten-Transformationstest.
- In vitro-Teste (betreffend humorale Immunantwort):
  Zweidimensionale Immunelektrophorese nach CLARKE, FREEMAN, RESSLER und LAURELL in der Modifikation nach OHLENSCHLÄGER,
  Immunglobuline quantitativ nach MANCINI;
  IgM; IgG, IgA.
- Akute-Phase-Proteine (quantitativ):
  Alpha-1-saures Glykoprotein,
  Alpha-1-Antitrypsin,
  Alpha-1-Antichymotrypsin,
  Alpha-2-Makroglobulin,
  Haptoglobin,
  Ceruloplasmin,
  C-reaktive protein.

Die ausgezeichnete Wirkung eines aus den Peyer'schen Plaques hergestellten Organextraktes auf die humorale Abwehr wurde in einer Anzahl von Versuchen bestätigt.

Ein Fall betraf eine 27-jährige Patientin, die seit 2 Jahren an juvenilem Diabetes litt. Durch eine ballaststoffreiche Kost (im Sinne von Burkitt), unterstützt durch das biologische Präparat Remedium Diabeticum und durch eine zusätzliche Injektion von 1 ml PPX-Z Extrakt - in der zweiten Woche 2 ml und in der dritten Woche 3 ml - konnte die tägliche Insulindosierung von 10 ml auf täglich 5 i.E. reduziert werden und die Blutzuckerwerte pendelten sich auf Werte um 94 mg% bis 100 mg% ein.

## Patentansprüche

1. Verwendung eines aus den Peyer'schen Plaques hergestellten Organextraktes zur Herstellung eines Arzneimittels zur unterstützenden Behandlung bei Diabetes mellitus sowie zur Behandlung von Pankreatitis.

2. Verwendung nach Anspruch 1, gekennzeichnet durch eine Verwendung in Verbindung mit Thymus-Organextrakt.

## Claims

1. Use of an organ extract produced from Peyer's patches for the manufacture of a medicament for supporting the treatment of diabetes mellitus and for the treatment of pankreatitis.

2. Use according claim 1, characterized by a use in connection with thymus organ extract.

## Revendications

1. Utilisation d'un extrait d'organe préparé à partir des plaques de Peyer pour l'obtention d'un médicament destiné au traitement de soutien dans le cas du diabète sucré ainsi qu'au traitement de la pancréatite.

2. Utilisation suivant la revendication 1, caractérisée par sa mise en oeuvre en liaison avec un extrait de thymus.